(19) Europäisches Patentamt
European Patent Office
Office européen des brevets

(11) Publication number: **0 507 272 B1**

(12) # EUROPEAN PATENT SPECIFICATION

(45) Date of publication of patent specification: **05.07.95**

(51) Int. Cl.$^6$: **A61K 7/06**

(21) Application number: **92105604.0**

(22) Date of filing: **01.04.92**

(54) **Hair cosmetic composition comprising a water soluble chitin derivative and a polyphenol.**

(30) Priority: **05.04.91 JP 73222/91**

(43) Date of publication of application:
**07.10.92 Bulletin 92/41**

(45) Publication of the grant of the patent:
**05.07.95 Bulletin 95/27**

(84) Designated Contracting States:
**AT CH DE FR GB LI NL**

(56) References cited:
**EP-A- 0 002 506**
**FR-A- 2 512 669**

**PATENT ABSTRACTS OF JAPAN vol. 12, no. 438 (C-544)(3285) 17 November 1988**

**PATENT ABSTRACTS OF JAPAN vol. 13, no. 182 (C-591)(3530) 27 April 1989**

**PATENT ABSTRACTS OF JAPAN vol. 7, no. 175 (C-179)(1320) 3 August 1983**

**PATENT ABSTRACTS OF JAPAN vol. 7, no. 135 (C-170)(1280) 11 June 1983**

(73) Proprietor: **Kao Corporation**
**14-10, Nihonbashi Kayabacho 1-chome**
**Chuo-Ku**
**Tokyo 103 (JP)**

(72) Inventor: **Imamura, Takashi**
**3-26-15-303 Natsumi**
**Funabashi-shi,**
**Chiba (JP)**
Inventor: **Liu, Wei Chen**
**3-8-C101 Makuharihongo**
**Chiba-shi,**
**Chiba (JP)**
Inventor: **Koyama, Takashi**
**1-3 Asahigaoka**
**Chiba-shi,**
**Chiba (JP)**

(74) Representative: **Kraus, Walter, Dr. et al**
**Patentanwälte Kraus, Weisert & Partner**
**Thomas-Wimmer-Ring 15**
**D-80539 München (DE)**

**EP 0 507 272 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a hair cosmetic composition, and, more particularly, to a hair cosmetic composition containing a water soluble chitin derivative and a polyphenol derivative or plant extract containing a polyphenol derivative. The hair cosmetic composition provides an excellent bounce, body and volume of the hair, and good styling manageability.

Soft hair or damaged hair does not have bounce, body, nor volume. It is difficult to set-up soft hair or damaged hair. Set-up or styling manageability is one of the most important points in the art of cosmetic treatment. Various kinds of cosmetic treatments have been developed and practiced. There are several methods to give hair an appropriate wave by using hair cosmetics, for example, a permanent hair treatment wave method, a temporal hair treatment by set lotion or hair spray. The permanent wave method tends to irreversibly damage healthy hair. Hair treatment by set-lotion or hair spray tends to become unset by humidity or water. These methods also give a stiff feeling to the hair.

Shampoo or conditioners containing a chitin derivative or hydrolyzed protein have been developed in order to improve styling manageability of the hair after washing the hair. But the effect is not yet sufficient.

It would be desirable, therefore, to provide a hair cosmetic composition which gives excellent bounce, body and volume, and good styling manageability to hair.

EP-A-0 002 506 relates to a hair cosmetic composition (hair conditioner) comprising a water soluble salt of chitosan.

FR-A-2 512 669 relates to hair cosmetic compositions comprising polyphenol derivatives such as phloroglucinol and tannin derivatives. Said cosmetic compositions are reported to provide good styling manageability of the hair.

Accordingly, it is an object of this invention to provide a hair cosmetic composition containing a water soluble chitin derivative which gives appropriate bounce, body and volume for good styling manageability of hair.

This and other objects of the invention which will become more apparent by the following description have been achieved by the use of a polyphenol derivative in combination with a water soluble chitin derivative. The resulting hair cosmetic composition gives appropriate bounce, body and volume enough for good styling manageability of the hair.

A hair cosmetic composition has been discovered which comprises: (A) 0.001 to 10% by weight of a water soluble chitin derivative, and (B) 0.00001 to 10% by weight of a polyphenol derivative.

The following chitin derivatives are used as chitin component (A) of the invention.

(1) Water soluble oligomers having more than one glucosamine monomer unit, which are obtained by degradation of chitin or chitosan. Suitable degradation methods include conventional methods of degradation, for example, the nitrous acid method, formic acid method, chlorine method, enzyme or microorganism methods, etc.

(2) Water soluble chitosan having 40-60% deacetylation ratio, which is obtained by controlling deacetylation of chitosan as described in Japanese laid-open patent application 53-47479/1978.

(3) Organic or inorganic salts of chitosan, for example, acetic acid salts, malic acid salts, citric acid salts, ascorbic acid salts, hydrochloric acid salts, sulfuric acid salts, phosphoric acid salts and the like.

(4) Water soluble chitin derivatives which are obtained by introducing a hydrophilic group into chitin or chitosan for example,

a) Polyoxyalkylene chitin or polyoxyalkylene chitosan having the following formula (I)

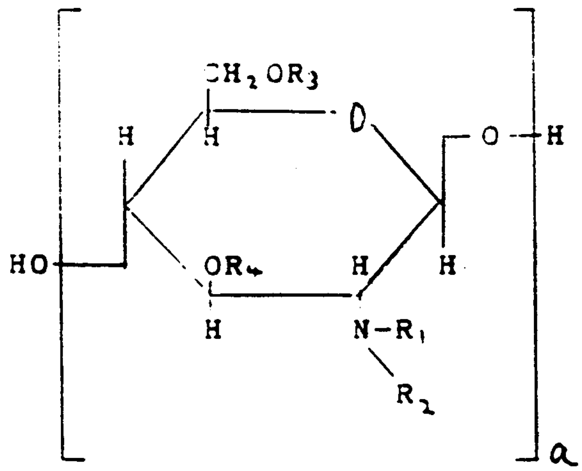

(I)

where a represents a number greater than one, $R_1$ and $R_2$ are H, $-COCH_3$ or $-(EO)_{p1}-(PO)_{q1}-(BO)_{r1}-H$, $R_3$ is H or $-(EO)_{p2}-(PO)_{q2}-(BO)_{r2}-H$, $R_4$ is H or $-(EO)_{p3}-(PO)_{q3}-(BO)_{r3}-H$, where $p_1$, $p_2$, $p_3$, $q_1$, $q_2$, $q_3$, $r_1$, $r_2$, $r_3$ are numbers from 0 to 5, $p_1 + q_1 + r_1 \neq 0$, $p_2 + q_2 + r_2 \neq 0$, $p_3 + q_3 + r_3 \neq 0$, EO represents an oxyethylene unit, PO represents an oxypropylene unit; BO represents an oxybutylene unit. The order of EO, PO, BO for bonding is not limited, so that PO may be bonded to the D-glucosamine unit and then the EO is bonded to the PO. Random bonding of EO, PO, BO is also possible. $R_1$, $R_2$, $R_3$, $R_4$, $p_1$, $p_2$, $p_3$, $q_1$, $q_2$, $q_3$, $r_1$, $r_2$, $r_3$, of each D-glucosamine unit may be independently selected.

b) Carboxymethyl chitin or carboxymethyl chitosan having following formula (II)

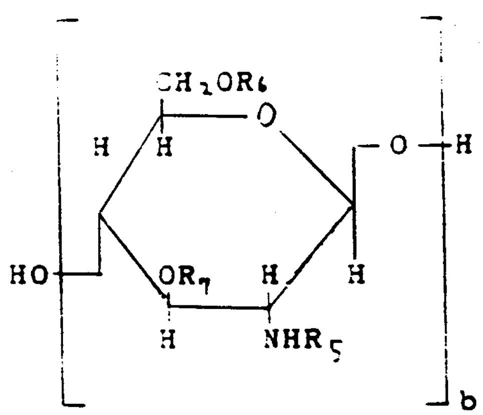

(II)

where b represents a number greater than one, $R_5$ is H or $-COCH_3$, $R_6$ and $R_7$ are H, $-CH_2COOH$, $-CH_2COOM$, where M is an alkali metal ion, or $-CH_2COO^-NH_4^+$; with the proviso that $R_6$ and $R_7$ are not a hydrogen atom at the same time. $R_5$, $R_6$, $R_7$ of each D-glucosamine unit may be independently selected.

c) Phosphoric esters of chitin or phosphoric esters of chitosan having following formula (III)

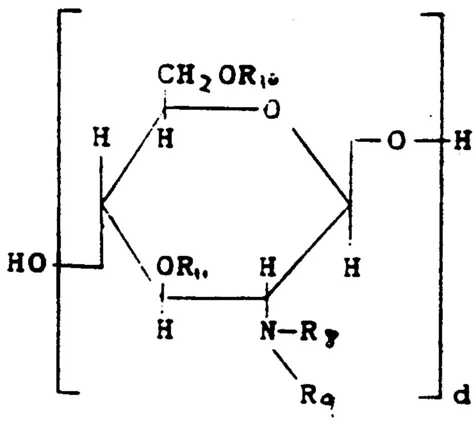

(III)

where d represents a number greater than one, $R_8$ is H or $-COCH_3$, $R_9$ is H or $-COCH_3$, $R_{10}$ is H or

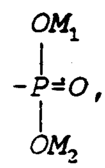

or $R_{11}$ is H or

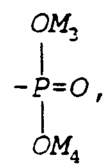

where $M_1$, $M_2$, $M_3$ and $M_4$ are H, alkali metal ions or ammonium ions; and $R_{10}$ and $R_{11}$ are not H at the same time. $R_8$, $R_9$, $R_{10}$ and $R_{11}$ of each D-glucosamine unit may be independently selected.

d) Sulfuric esters of chitin or sulfuric esters of chitosan having the following formula (IV)

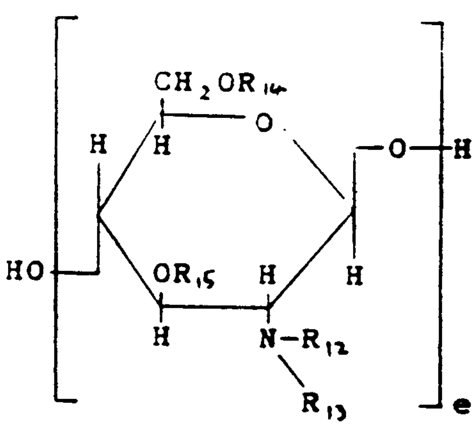

(IV)

where e represents a number greater than one, $R_{12}$ is H or $-COCH_3$, $R_{13}$ is H or $-COCH_3$, $R_{14}$ is H or

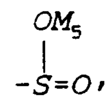

$R_{15}$ is H or

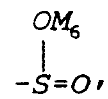

where $M_5$ and $M_6$ are H, alkali metal or ammonium ions, and $R_{12}$, $R_{13}$, $R_{14}$ and $R_{15}$ of each D-glucosamine unit may be independently selected.

e) Dihydroxypropyl chitin or dihydroxypropyl chitosan having the following formula (V)

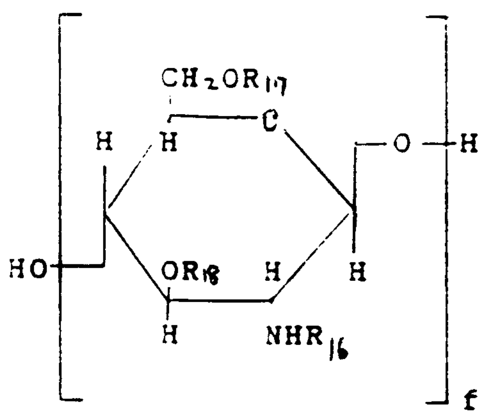

(V)

where f represents a number greater than one, $R_{16}$ is H, $-COCH_3$ or $-CH_2CH(OH)-CH_2OH$, and $R_{17}$ and $R_{18}$ are H or $-CH_2CH(OH)CH_2OH$, $R_{17}$ and $R_{18}$ are not H at the same time.

f) N-2-hydroxypropyl sulfonic acid chitosan having the following formula (VI)

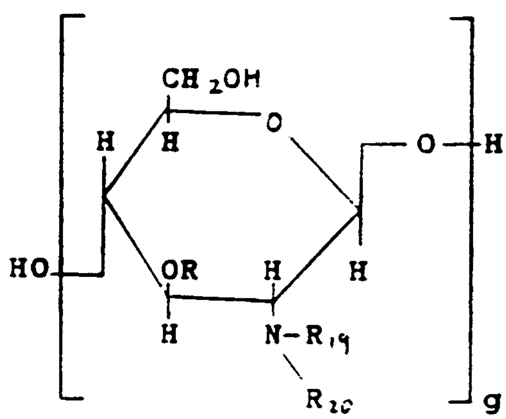

(VI)

where g represents a number greater than one, $R_{19}$ is H,

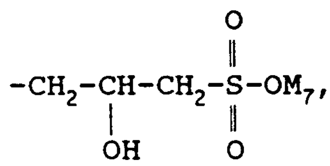

$R_{20}$ is H or

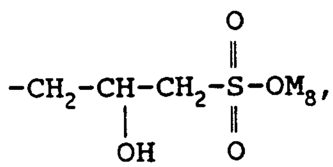

wherein $M_7$ and $M_8$ are alkali metal or ammonium ions; and $R_{19}$ and $R_{20}$ are not H at the same time.

Water soluble chitin derivatives may be obtained by the following known methods:

(1) reacting chlorohydroxypropylene, chlorohydroxybutylene, ethyleneoxide, propyleneoxide, or butyleneoxide with polyoxyalkylene chitin or polyoxyalkylene chitosan under ambient temperature and pressure conditions or under conditions of 50-60 °C and 1-5Kg/cm pressure; or

(2) reacting chitin or chitosan with an alkyleneoxide in an organic solvent/water mixture, under alkaline conditions. See Japanese 64-5601/1989.

Carboxyl chitin or carboxyl chitosan can be obtained by the reaction of alkali-chitin or alkali-chitosan with monochloroacetic acid.

Phosphoric esters of chitin or phosphoric esters of chitosan can be obtained by the reaction of chitin or chitosan dissolved or dispersed in methane sulfonate and phosphorus pentoxide with cooling of the reactant. This method is described in, for example, Japan Chemical Society 48th autumn meeting Preprint II, p. 570.

Sulfuric acid esters of chitin or sulfuric acid esters of chitosan are obtained by reaction of chitin or chitosan activated in pyridine with $SO_3$-pyridine complex. See M.L. Volform et al., The Sulfonation of Chitosan, J. Am. Chem. Soc., vol 81, pp. 1764-1766, 1959.

Dihydroxypropyl chitin or dihydroxypropyl chitosan are obtained by reaction of epichlorhydrin with alkali-chitin or alkali-chitosan under conditions of high temperature.

N-2-hydroxypropyl sulfonic acid chitosan is obtained by reaction of glycidyl sulfonic acid with chitosan at high temperature, high pressure and in the presence of an alkali catalyst.

Preferably, polyoxyalkylene chitin or polyoxyalkylene chitosan is used for the hair cosmetic composition of this invention. More preferably, chitin derivatives having more than three (3) alkyleneoxide units per glucosamine structure is used for the hair cosmetic composition of the invention to give good bounce, body and volume of the hair. Chitin derivatives having more than four (4) moles of alkyleneoxide per glucosamine structure are most preferred.

Water soluble chitin derivative (A) is preferably used in the hair cosmetic composition of this invention in an amount of 0.001-10% by weight, preferably 0.01-5% by weight. The use of the water soluble chitin derivative at less than 0.001% by weight may result in an insufficiency of bounce, body and volume of the hair. The presence of the water soluble chitin derivative at more than 10% by weight may result in a stiff feeling of the hair.

Polyphenol derivative (B) of this invention includes compounds having more than two phenolic hydroxyl groups and derivatives thereof. It includes phloroglucinol derivatives, e.g., phloroglucinol, aspidin, aspidinol, and tannin derivatives, e.g., tannin, pyrogallol tannin, catechol tannin. Tannin derivatives are most preferable for this invention.

Polyphenol derivative (B) of this invention is used in an amount of 0.00001-10% by weight, preferably 0.0001-1% by weight. If the amount of component (B) is less than 0.00001% by weight, the bounce, body and volume of the hair are insufficient. If the amount of component (B) is more than 10%, the hair may become stiff.

Crude extracts of plants or extracts of plants containing polyphenol derivatives are used as the component (B) of the invention. Crude extracts containing tannin obtained from mimosa, quebracho, gambir-catechu, Japanese gall, nut gall, aleppo gall, Turkey gall, are preferred for use in the invention.

Component (B) of the present invention is obtained by extraction of plants. Examples of suitable plants include birch, rosemary, arnica, hamamelis, camomile, sage, St. John's bread, henna, hop, lime, aloe, wild thyme, calendula, horsetail, mountain gentian, nettle, chestnut, avocado, seaweed, milfoil, coltsfoot, marigold, peach, rose, senna, thyme, and white lily. Of these plants, birch, rosemary, hamamelis, camomile, sage, aloe, henna, and St. John's bread are preferable and most preferably mentioned are birch and rosemary.

Suitable parts of these preferred plants for the extraction are, for example, the bark of birch, the entire grass part of rosemary, the leaves of hamamelis, the leaves of sage, the leaves of aloe, the leaves of henna, and the fruit of St. John's bread.

The plant extract is obtained by extracting flowers, leaves, fruits, roots, stems and the like with solvent at ambient temperature or with heating according to any known extraction technique. The extraction solvents are preferably polar organic solvents including, for example, lower alcohols ($C_{1-6}$) such as methanol, ethanol and the like, $C_{2-6}$ alkylene glycols such as propylene glycol, 1,3-butylene glycol and glycerin, and water. These solvents may be used singly or in combination.

The extracts of birch and rosemary which are the most preferable plants are commercially available. The commercially sold birch extracts include Birch Extract made by Novarom Co., Ltd., Boulene MCF787 by Gattefosse Co., Ltd., Birch Extract by Virmin Co., Ltd., Birch Extract by Nowak Co., Ltd., Extrapone Birch Special by Dragoco Co., Ltd., and the like. Suitable as rosemary extracts are Rosemary Extract by Novarom Co., Ltd., Romarin MCF772 by Gattefosse Co., Ltd., Phytelene EG009 by Virmin Co., Ltd., and Rosemary Extract by Nowak Co., Ltd.

When the plant extract is in a liquid form, component (B) is used in an amount ranging from 0.001 to 5% by weight, preferably 0.01 to 1.0% by weight of the total composition, calculated as a residue obtained after distillation of extraction solvent. If the amount of the plant extract is less than 0.001% by weight, the effect of the invention is insufficient. If the amount of the plant extract is more than 5% by weight, the hair may become stiff.

The component (B) or plant extract may be directly added to hair cosmetic compositions in the form of a liquid extract, may be added after concentration to a desired level or after complete removal of extraction solvent from the extract.

Besides the two essential components, various components commonly known for use in a cosmetic composition, detergent composition, medical composition or food, can be formulated to the extent that the effects of the present invention are not adversely affected. Such optional components include, for example, an anionic surfactant, e.g., alkylsulfate, polyoxyethylene alkylsulfate, alpha-olefin alkylsulfate, sulfosuccinic acid half ester, acyl glutamate, monoalkyl phosphate, soap; amphoteric surfactant, e.g., amidoamino acid, alkylbetaine; cationic surfactant, e.g., alkyl quaternary ammonium salt; fats and oils, e.g., silicone derivatives, perfluoropolyether, ester oils, higher alcohol; water; moisture retaining agent, e.g., glycerin, propylene glycol; pharmaceutical component, e.g., anti-inflammatory agent, anti-dandruff agent, vitamin; antiseptics, e.g., hydroxybenzoic acid, urea; thickening agent, e.g., water soluble polymer; UV absorber, e.g., oxybenzone; antioxidation agent, e.g., dibutyl hydroxytoluene; tocopherol acetic acid ester; coloring agent, e.g., dyestuff, pigment; conditioning agent, e.g., cationic polymer; pearl like component, e.g., glycol ester; hair setting polymer such as acryl resin; perfumes; and component disclosed in ENCYCLOPEDIA OF CONDITIONING RINSE INGREDIENTS (MICELLE PRESS, 1987).

The hair cosmetic composition of the present invention can be prepared in liquid, paste, solid, powder, cream, aerosol or any other forms, according to conventional methods by using component (A) and (B). The term "hair cosmetic composition" includes hair treatment compositions, shampoo compositions, hair rinse compositions, after shampoo compositions, hair conditioners, set lotions, blow styling lotions, hair sprays, hair dyes, bleaches, permanent wave 1st agents, permanent wave 2nd agents, hair coloring agents, hair liquids, and hair tonics.

Other features of the invention will become apparent in the course of the following descriptions of exemplary embodiments which are given for illustration of the invention and are not intended to be limiting thereof.

## Examples

### Example 1

Shampoo compositions were prepared according to the formulations listed in Table 1. Each composition was tested in terms of its bounce, body and volume after washing and drying.

Evaluation method and standard

In the evaluation, 1g of sample composition was applied to a bundle of hair (Weight: 20g, length: 15cm) of a healthy Japanese women. Bounce, body and volume of the hair were determined after the shampoo was foamed for 1 minute, rinsed and dried by hair drier. Bounce, body and volume of the hair were evaluated by 10 expert panelists according the following standards.

Bounce, body of the hair

AA - substantial bounce and body of the hair
BB - moderate bounce and body of the hair
CC - slight bounce and body of the hair
DD - poor bounce and body of the hair

Volume of the hair

AA - substantial volume of the hair
BB - moderate volume of the hair
CC - slight volume of the hair
DD - poor volume of the hair

Table 1

| COMPONENTS (% by weight) | Invention Compositions | | | Comparative Compositions | | |
|---|---|---|---|---|---|---|
| | 1 | 2 | 3 | A | B | C |
| Sodiumm polyoxyethylene(3)-dodecylether sulfate | 10 | 0 | 0 | 10 | 0 | 0 |
| Laurylphosphate triethanolamine | 0 | 10 | 0 | 0 | 10 | 0 |
| Sodium laurylsulfosuccinate | 0 | 0 | 10 | 0 | 0 | 10 |
| Polyoxyethylene-polyoxypropylene chitosan*1 | 0 | 0 | 0.3 | 0 | 0 | 0 |
| Polyoxypropylene chitosan*2 | 0.2 | 0.2 | 0 | 0 | 0.2 | 0 |
| Rosemary extract*3 | 0.3 | 0 | 0.2 | 0.3 | 0 | 0.2 |
| Camomile extract*4 | 0 | 0.2 | 0 | 0 | 0 | 0 |
| Deionized water | balance | balance | balance | balance | balance | balance |
| Bounce, body of the hair | AA | AA | AA | DD | CC | DD |
| Volume of the hair | AA | AA | AA | DD | CC | DD |

*1 average molar number of EO = 4.0
average molar number of PO = 4.0

*2 average molar number of PO = 4.0

*3 containing 1% of polyphenol derivatives

*4 containing 0.3% of polyphenol derivatives

Examples 2-5

A shampoo composition (Examples 2 and 6), hair rinse composition (Example 3), hair treatment composition (Example 4), and pretreatment composition (Example 5) were prepared. These compositions gave hair good bounce, body and volume, after drying.

Example 2 - Shampoo composition

| | |
|---|---|
| Polyoxyethylene(3)dodecylethersulfate Triethanol amine | 10 wt % |
| Lauryl sulfate triethanol amine | 4 |
| Coconut oil fatty acid diethanol amide | 2 |
| Cationic polymer (JR-400, product of UCC) | 0.3 |
| Polyoxyethylene chitosan (*5) | 1 |
| Birch extract | 1 |
| Sodium chloride | 1 |
| Antiseptics | 0.1 |
| Coloring agent, flavor, pH adjusting agent | Effective Amount |
| Water | Balance |
| | 100 wt % |

*5-average molar number of EO = 3.0.

Example 3 - Hair rinse composition

| | |
|---|---|
| Stearyl trimethyl ammonium chloride | 2 wt % |
| Dialkyl dimethyl ammonium chloride(*6) | 0.5 |
| Cetanol | 4 |
| Liquid paraffin | 0.1 |
| Dimethylpolysiloxane | 0.3 |
| Isostearic acid | 0.1 |
| Hydroxyethyl cellulose(HEC SE850, product of Dycel) | 0.3 |
| Propylene glycol | 5 |
| Polyoxypropylene chitosan (*7) | 1 |
| Rosemary extract | 1 |
| Flavor | 0.5 |
| Coloring agent, pH adjusting agent | Effective Amount |
| Water | Balance |
| | 100 wt % |

*6 Branched quaternary ammonium salt obtained from commercially available oxoalcohol having $C_{12-15}$ (mixture of DOBANOL 23 and 45, product of Mitsubishi Petrochemical Co., Ltd.) branch ratio is 20%
*7-average molar number of PO = 4.0.

Example 4 - Hair treatment composition

| | |
|---|---|
| Cetyltrimethylammonium chloride | 3.0 wt % |
| 2-Dodecylhexadecyl trimethyl ammonium chloride | 2.0 |
| Cetyl alcohol | 3.0 |
| Stearyl alcohol | 2.0 |
| Solid paraffin | 1.0 |
| Behenic acid | 1.0 |
| Myristyl myristate | 0.5 |
| Collagen hydrolyzed product | 1.0 |
| Amodimethicone emulsion** | 1.0 |
| Polyoxyethylene polyoxypropylene chitosan (*8) | 5.0 |
| Dipropylene glycol monoethylether | 20.0 |
| Birch extract | 3.0 |
| Flavor | 1.0 |
| Coloring agent | Effective Amount |
| Water | Balance |
| | 100 wt % |

**-Modified silicone polymer emulsion SM8702C (containing 40% by weight of amodimethicone, product of Toray-Dow Corning).
*8-average molar number of EO = 2.0; average molar number of PO = 2.0.

Example 5 - Pretreatment Composition

| | |
|---|---|
| Behenyltrimethylammonium chloride | 0.5 wt % |
| Cetyl trimethyl ammonium chloride | 1.0 |
| Coconut oil fatty acid diethanol amide | 1.0 |
| Polyoxyethylene hydrogenated caster oil | 1.0 |
| Avocado oil | 0.1 |
| Polyether modified silicone (KF353A) | 0.2 |
| Polyoxypropylene chitosan (*9) | 3.0 |
| Silk protein | 0.5 |
| Poly dimethyldiaryl ammonium chloride (MERQUAT 100) | 0.5 |
| Aloe extract | 1.0 |
| Hydroxy propylmethyl cellulose (METOLOSE 60 SH4000) | 1.0 |
| Camomile extract | 1.0 |
| Paraben (methyl paraben) | 0.1 |
| Ethanol | 5.0 |
| Glycerin | 5.0 |
| Flavor | 0.3 |
| Coloring agent, pH adjusting agent | Effective Amount |
| Water | Balance |
| | 100 wt % |

*9-average molar number of PO = 2.0.

Example 6 - Shampoo composition

| | |
|---|---|
| Lauryl sulfate triethanol amine | 15 wt % |
| Coconut oil fatty acid diethanol amide | 3 |
| Carboxymethyl chitin (Chitin liquid, act. 0.2%, product of Ichimaru - Farcos) | 2 |
| Catechol tannin | 0.001 |
| Hydroxypropyl methyl cellulose (METOROSE 60-SH-400, product of Shin-Etsu Chemical) | 1 |
| Coloring agent, pH adjusting agent | Effective Amount |
| Water | Balance |
| | 100 wt % |

The hair cosmetic composition of this invention provides hair with good bounce, body and volume and good hair styling manageability, and is useful as a hair treatment, hair conditioner, shampoo or rinse.

## Claims

**1.** A hair cosmetic composition, comprising:
(A) a water soluble chitin derivative, and
(B) a polyphenol derivative.

**2.** The hair cosmetic composition according claim 1, comprising 0.001-10% by weight of said water soluble chitin derivative.

**3.** The hair cosmetic composition according claim 1, comprising 0.01-5% by weight of said water soluble chitin derivative.

4. The hair cosmetic composition according claim 1, comprising 0.1-1% by weight of said water soluble chitin derivative.

5. The hair cosmetic composition according claim 1, comprising 0.00001-10% by weight of said polyphenol derivative.

6. The hair cosmetic composition according claim 1, comprising 0.0001-1% by weight of said polyphenol derivative.

7. The hair cosmetic composition according claim 1, wherein said water soluble chitin derivative is a polyoxyalkylene chitin or polyoxyalkylene chitosan.

8. The hair cosmetic composition according claim 7, wherein said water soluble chitin derivative is a polyoxyalkylene chitin or polyoxyalkylene chitosan having more than three oxyalkylene units per glucosamine unit of the chitin derivative.

9. The hair cosmetic composition according claim 1, wherein said polyphenol derivative is selected from the group consisting of a phloroglucinol derivatives and tannin derivatives.

10. The hair cosmetic composition according claim 9, wherein said polyphenol derivative is a tannin derivative.

11. The hair cosmetic composition according claim 1, wherein said polyphenol derivative is a plant extract containing a polyphenol derivative.

12. The hair cosmetic composition according claim 11, wherein said plant extract is selected from the group consisting of a birch extract, a rosemary extract, a hamamelis extract, a camomile extract, a sage extract, an aloe extract, a henna extract, and a St. John's bread extract.

13. The hair cosmetic composition according claim 12, wherein said plant extract is a birch extract or a rosemary extract.

14. The hair cosmetic composition according claim 11, comprising 0.001-5.0% by weight of said plant extract.

15. The hair cosmetic composition according claim 14, comprising 0.01-1.0% by weight of said plant extract.

16. The hair cosmetic composition according claim 14, comprising 0.1-0.5% by weight of said plant extract.

17. A method for improving the bounce, body and volume of hair comprising contacting hair with a hair cosmetic composition comprising:
   (A) a water soluble chitin derivative and
   (B) a polyphenol derivative.

**Patentansprüche**

1. Haarkosmetikum, enthaltend
   (A) ein wasserlösliches Chitinderivat, und
   (B) ein Polyphenolderivat.

2. Haarkosmetikum nach Anspruch 1, dadurch **gekennzeichnet,** daß es 0,001-10 Gew.-% des wasserlöslichen Chitinderivats enthält.

3. Haarkosmetikum nach Anspruch 1, dadurch **gekennzeichnet,** daß es 0,01-5 Gew.-% des wasserlöslichen Chitinderivats enthält.

**4.** Haarkosmetikum nach Anspruch 1, dadurch **gekennzeichnet,** daß es 0,1-1 Gew.-% des wasserlöslichen Chitinderivats enthält.

**5.** Haarkosmetikum nach Anspruch 1, dadurch **gekennzeichnet,** daß es 0,00001-10 Gew.-% des Polyphenolderivats enthält.

**6.** Haarkosmetikum nach Anspruch 1, dadurch **gekennzeichnet,** daß es 0,0001-1 Gew.-% des Polyphenolderivats enthält.

**7.** Haarkosmetikum nach Anspruch 1, dadurch **gekennzeichnet,** daß das wasserlösliche Chitinderivat ein Polyoxyalkylenchitin oder ein Polyoxyalkylenchitosan ist.

**8.** Haarkosmetikum nach Anspruch 7, dadurch **gekennzeichnet,** daß das wasserlösliche Chitinderivat ein Polyoxyalkylenchitin oder Polyoxyalkylenchitosan mit mehr als drei Oxyalkyleneinheiten pro Glucosamineinheit des Chitinderivats ist.

**9.** Haarkosmetikum nach Anspruch 1, dadurch **gekennzeichnet,** daß das Polyphenolderivat ausgewählt ist aus der Gruppe bestehend aus Phloroglucinderivaten und Tanninderivaten.

**10.** Haarkosmetikum nach Anspruch 9, dadurch **gekennzeichnet,** daß das Polyphenolderivat ein Tanninderivat ist.

**11.** Haarkosmetikum nach Anspruch 1, dadurch **gekennzeichnet,** daß das Polyphenolderivat ein Pflanzenextrakt ist, der ein Polyphenolderivat enthält.

**12.** Haarkosmetikum nach Anspruch 11, dadurch **gekennzeichnet,** daß der Pflanzenextrakt ausgewählt ist aus der Gruppe bestehend aus einem Birkenextrakt, einem Rosmarinextrakt, einem Hamamelisextrakt, einem Kamillenextrakt, einem Salbeiextrakt, einem Aloeextrakt, einem Hennaextrakt und einem Johannisbrotextrakt.

**13.** Haarkosmetikum nach Anspruch 12, dadurch **gekennzeichnet,** daß der Pflanzenextrakt ein Birkenextrakt oder ein Rosmarinextrakt ist.

**14.** Haarkosmetikum nach Anspruch 11, dadurch **gekennzeichnet,** daß es 0,001-5,0 Gew.-% des Pflanzenextrakts enthält.

**15.** Haarkosmetikum nach Anspruch 14, dadurch **gekennzeichnet,** daß es 0,01-1,0 Gew.-% des Pflanzenextrakts enthält.

**16.** Haarkosmetikum nach Anspruch 14, dadurch **gekennzeichnet,** daß es 0,1-0,5 Gew.-% des Pflanzenextrakts enthält.

**17.** Verfahren zur Verbesserung der Elastizität, der Fülle und des Volumens von Haar, dadurch **gekennzeichnet,** daß man das Haar mit einem Haarkosmetikum in Kontakt bringt, das
(A) ein wasserlösliches Chitinderivat, und
(B) ein Polyphenolderivat enthält.

**Revendications**

**1.** Composition cosmétique pour les cheveux comprenant :
(A) un dérivé de chitine hydrosoluble, et
(B) un dérivé polyphénol.

**2.** Composition cosmétique pour les cheveux selon la revendication 1, comprenant 0,001 à 10 % en poids, dudit dérivé de chitine hydrosoluble.

**3.** Composition cosmétique pour les cheveux selon la revendication 1, comprenant 0,01 à 5 % en poids, dudit dérivé de chitine hydrosoluble.

4. Composition cosmétique pour les cheveux selon la revendication 1, comprenant 0,1 à 1 % en poids, dudit dérivé de chitine hydrosoluble.

5. Composition cosmétique pour les cheveux selon la revendication 1, comprenant 0,00001 à 10 % en poids, dudit dérivé polyphénol.

6. Composition cosmétique pour les cheveux selon la revendication 1, comprenant 0,0001 à 1 % en poids, dudit dérivé polyphénol.

7. Composition cosmétique pour les cheveux selon la revendication 1, dans laquelle ledit dérivé de chitine hydrosoluble est une polyoxyalkylène chitine ou un polyoxyalkylène chitosane.

8. Composition cosmétique pour les cheveux selon la revendication 7, dans laquelle ledit dérivé de chitine hydrosoluble est une polyoxyalkylène chitine ou un polyoxyalkylène chitosane comportant plus de trois unités oxyalkylène par unité de glucosamine du dérivé de chitine.

9. Composition cosmétique pour les cheveux selon la revendication 1, dans laquelle dedit dérivé polyphénol est choisi dans le groupe comprenant les dérivés de phloroglucinol, et les dérivés de tanin.

10. Composition cosmétique pour les cheveux selon la revendication 9, dans laquelle ledit dérivé de polyphénol est un dérivé du tanin.

11. Composition cosmétique pour les cheveux selon la revendication 1, dans laquelle ledit dérivé de polyphénol est un extrait de plante contenant un dérivé de polyphénol.

12. Composition cosmétique pour les cheveux selon la revendication 11, dans laquelle ledit extrait de plante est choisi dans le groupe comprenant un extrait de bouleau, un extrait de romarin, un extrait d'hamamélis, un extrait de camomille, un extrait de sauge, un extrait d'aloès, un extrait de henné, et un extrait de caroube.

13. Composition cosmétique pour les cheveux selon la revendication 12, dans laquelle ledit extrait de plante est un extrait de bouleau, ou un extrait de romarin.

14. Composition cosmétique pour les cheveux selon la revendication 11, comprenant 0,001 à 5,0 % en poids dudit extrait de plante.

15. Composition cosmétique pour les cheveux selon la revendication 14, comprenant 0,01 à 1,0 % en poids dudit extrait de plante.

16. Composition cosmétique pour les cheveux selon la revendication 14, comprenant 0,1 à 0,5 % en poids dudit extrait de plante.

17. Méthode pour améliorer la vigueur, la masse, et le volume des cheveux, comprenant la mise en contact des cheveux avec une composition cosmétique pour les cheveux comprenant :
   (A) un dérivé de chitine hydrosoluble, et
   (B) un dérivé polyphénol.